(19) Europäisches Patentamt
European Patent Office
Office européen des brevets

(11) Publication number: 0 049 554 A2

(12) # EUROPEAN PATENT APPLICATION

(21) Application number: 81201190.6

(22) Date of filing: 03.12.79

(51) Int. Cl.³: C 07 D 213/71

(30) Priority: 04.12.78 US 966258
22.10.79 US 83753

(43) Date of publication of application:
14.04.82 Bulletin 82/15

(84) Designated Contracting States:
BE DE FR GB IT LU NL

(60) Publication number of the earlier application in accordance with Art. 76 EPC: 0 013 480

(71) Applicant: E.I. DU PONT DE NEMOURS AND COMPANY
Legal Department 1007 Market Street
Wilmington Delaware 19898(US)

(72) Inventor: Levitt, George
3218 Romilly Road Cardiff
Wilmington Delaware 19810(US)

(74) Representative: Hildyard, Edward Martin et al,
Frank B. Dehn & Co. European Patent Attorneys Imperial House 15-19 Kingsway
London WC2B 6UZ(GB)

(54) **Substituted pyridinesulfonylisocyanates and preparation thereof.**

(57) Substituted pyridinesulfonylisocyanates of the formula

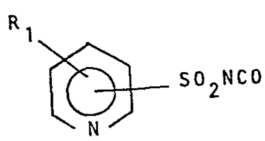

wherein $R_1$ is halogen, $CH_3$, $NO_2$ or alkoxycarbonyl are valuable intermeidates for the preparation of herbicidal pyridinesulfonylurea derivatives. They may be prepared by reacting a corresponding $R_1$-substituted N-(alkylaminocarbonyl)pyridinesulfonamide with phosgene.

EP 0 049 554 A2

"Substituted pyridinesulfonylisocyanates and preparation thereof"

This invention relates to substituted pyridinesulfonylisocyanates and their preparation.

The compounds of this invention have the general formula

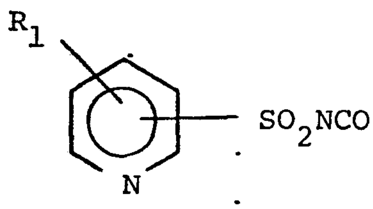

(I)

wherein $R_1$ is F, Cl, Br, $CH_3$, $CO_2R_5$ or $NO_2$; and $R_5$ is $C_1$-$C_3$ alkyl:

Preferred compounds are those wherein the sulfonylisocyanate group is at the 3-position of the pyridine ring. Within this group of compounds we further prefer those wherein $R_1$ is at the 2- or 4-position of the pyridine ring. In this latter group of compounds we still further prefer those wherein $R_1$ is F, Cl or Br, and especially 2-fluoro-, 2-chloro- and 2-bromo-3-pyridinesulfonylisocyanates.

The compounds of formula (I) are novel and valuable chemical intermediates. They may, for instance, be reacted with an appropriately substituted aminopyrimidine or aminotriazine to yield pyridinesulfonylurea derivatives having potent herbicidal and plant growth regulant activities (see our copending European Patent Application No. 79302769.9, published as Specification No. 13480, to which the reader is referred for a full disclosure of the utility of these compounds). The preferences expressed above arise as a consequence of the superior activity and/or cost of the pyridinesulfonylureas derivable from the preferred compounds of formula (I).

The compounds of formula (I) can be made by reacting a corresponding $R_1$-substituted N-(alkylaminocarbonyl)-

pyridinesulfonamide with phosgene. The reaction can be performed in a refluxing solvent such as xylene or chlorobenzene followed by filtration of the solution at room temperature and removal of the solvent. An exemplary reaction is illustrated below:

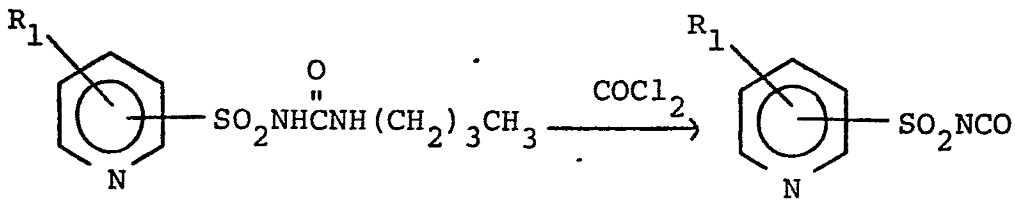

(II) (I)

The N-(alkylaminocarbonyl)pyridinesulfonamides of formula (II) may be conveniently prepared by the reaction of equivalent molar amounts of an appropriately substituted pyridinesulfonamide, an alkyl isocyanate and an anhydrous base such as $K_2CO_3$ or $CaCO_3$ in an anhydrous solvent such as acetone or acetonitrile. Addition of water and adjustment of the pH to about 3 with HCl, followed by filtration yields the desired N-(alkylaminocarbonyl)-pyridinesulfonamides.

The synthesis of sulfur compounds of pyridine has been reviewed in "The Chemistry of Heterocyclic Compounds", a series published by Interscience Publ., New York and London. Pyridinesulfonamides are described by H. L. Tale in "Pyridine and Its Derivatives" Supplement, Part 4 (1975), to which the reader is referred.

The compounds of this invention and their preparation are further illustrated in the following Example.

<u>Example</u>

<u>2-Chloro-3-pyridinesulfonylisocyanate</u>

To 125 ml of dry xylene was added with stirring 20.7 g of 2-chloro-N-(butylcarbamoyl)-3-pyridinesulfonamide. This solution was heated to reflux, and phosgene added until no further uptake of this gas was observed. It was then cooled, filtered and the solvent was removed

*in vacuo* to yield 2-chloro-3-pyridinesulfonylisocyanate as an oil, Bp 108-110$^{o}$C (0.7 mm Hg). This product shows a sharp absorption peak in the infrared region at 2220 $cm^{-1}$.

Using the above procedure and the appropriate N-(alkylaminocarbonyl)-pyridinesulfonamide, one may likewise prepare the sulfonylisocyanates in the following Table.

Table

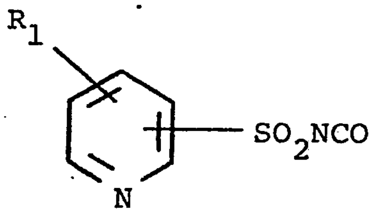

| $R_1$ | Position $-SO_2NCO$ |
|---|---|
| 6-Cl | 3 |
| $5-CH_3$ | 3 |
| $6-CH_3$ | 3 |
| $4-CO_2C_2H_5$ | 3 |
| $2-CO_2CH_3$ | 3 |
| $4-CO_2iC_3H_7$ | 3 |
| 4-Cl | 3 |
| 2-F | 3 |
| 2-Br | 3 |
| 4-F | 3 |
| 4-Br | 3 |
| 3-Br | 4 |
| $3-CH_3$ | 4 |
| $2-NO_2$ | 3 |
| $2-CO_2-\underline{i}-C_3H_7$ | 3 |
| $2-CO_2CH_3$ | 3 |
| $2-CH_3$ | 3 |
| $4-NO_2$ | 3 |
| $4-CH_3$ | 3 |
| $4-CO_2CH_3$ | 3 |
| $4-CO_2-\underline{i}-C_3H_7$ | 3 |
| 3-Cl | 4 |
| $3-NO_2$ | 4 |
| $3-CH_3$ | 4 |
| $3-CO_2CH_3$ | 4 |
| $3-CO_2-\underline{i}-C_3H_7$ | 4 |

Claims:-

1. Compounds having the formula

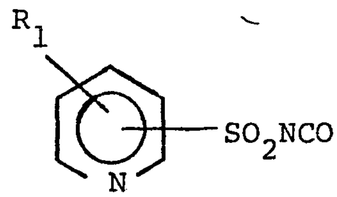

(I)

wherein $R_1$ is F, Cl, Br, $CH_3$, $CO_2R_5$ or $NO_2$; and $R_5$ is $C_1$-$C_3$ alkyl.

2. Compounds of claim 1 wherein the sulfonylisocyanato group is at the 3-position of the pyridine ring.

3. Compounds of claim 2 wherein $R_1$ is at the 2- or 4-position of the pyridine ring.

4. Compounds of claim 3 wherein $R_1$ is at the 2-position of the pyridine ring.

5. Compounds of claim 3 wherein $R_1$ is F, Cl or Br.

6. A compound of claim 1 which is 2-chloro-3-pyridinesulfonylisocyanate.

7. A compound of claim 1 which is 2-fluoro-3-pyridinesulfonylisocyanate.

8. A compound of claim 1 which is 2-bromo-3-pyridinesulfonylisocyanate.

9. A process for making a compound of any of claims 1 to 7 which comprises reacting a corresponding $R_1$-substituted N-(alkylaminocarbonyl)-pyridinesulfonamide with phosgene.